# EUROPEAN PATENT APPLICATION

(11) **EP 4 520 397 A1**
(43) Date of publication of application: **12.03.2025**
(21) Application number: 24191583.4
(22) Date of filing: 29.07.2024
(51) Int. Cl.: A61P 35/00, A61K 38/17, C07K 14/47, C12N 15/62

(54) **POLYPEPTIDES COMPRISING HISTONE CODE READER DOMAINS AND USES THEREOF**

(30) Priority: 28.07.2023 KR 20230098780
(71) Applicant: Korea Advanced Institute of Science and Technology, Daejeon 34141 (KR); Ajou University Industry-Academic Cooperation Foundation, Suwon-si, Gyeonggi-do 16499 (KR)
(72) Inventor: LEE, Ji Min, 34141 Daejeon (KR); YOO, Tae Hyeon, 17003 Yongin-si (KR); GWON, Minji, 16499 Suwon-si (KR); PARK, SU CHAN, 34141 Daejeon (KR); PARK, Il-Geun, 34141 Daejeon (KR); PARK, Sangah, 34141 Daejeon (KR); LEE, Sangwoo, Jeju-si (KR)
(74) Representative: Mewburn Ellis LLP

(57) **Abstract**

The present invention relates to a polypeptide comprising a histone code reader domain and uses thereof, in particular to a polypeptide comprising the PHD domain of PHF6 (PHD finger protein 6), optionally DPF3b (double PHD fingers 3b) or BPTF (bromodomain PHD finger transcription factor) or variants thereof, and uses thereof for the prevention or treatment of cancer.

## Description

### [Technical Field]

The present invention relates to a polypeptide comprising a histone code reader domain and use thereof, and more particularly to a polypeptide comprising the PHD domain of PHF6 (PHD finger protein 6), optionally DPF3b (double PHD fingers 3b), or variants thereof, and use thereof for the prevention or treatment of cancer.

### [Related Art]

In eukaryotic cells, DNA is surrounded by proteins called histones, which protect and regulate the DNA. The DNA structure surrounded by histones is called chromatin and plays an important role in regulating gene expression. The ability of eukaryotic cells to maintain distinct phenotypes despite containing the same genetic component is ensured by various chemical modifications in chromatin-associated proteins, DNA and histones.

Epigenetics refers to genetic changes that occur in the genome that result in altered patterns of gene expression without affecting the core DNA sequence. Even though these changes do not affect the basic DNA sequence, they play an important role in the identity of the cell and regulate important cellular processes. Chromatin in the cell nucleus exists in two states: closed chromatin (heterochromatin), which is associated with transcriptional repression, and open chromatin (euchromatin), which favors transcription. This level of chromatin regulation is highly dependent on post-translational modifications of core histone proteins and covalent modifications of DNA.

Nucleosome, the basic building block of chromatin, is composed of octamer of histone protein (heterodimer of H3 and H4 and heterodimer of H2A and H2B) that forms a dense central structure that wraps around 147 base pairs of DNA. Histone protein has an N-terminal tail that protrudes from the center and undergoes various post-translational modifications. These modifications, along with direct modifications of DNA, constitute the epigenetic code (Epigenetic mediation of environmental influences in major psychotic disorders, Schizophr Bull. 2009 Nov;35(6):1045-56).

A distinctive feature of these code is their ability to adapt and respond to environmental changes that occur during the life cycle of an organism which grows from a zygote to an adult. Four types of cytosine residue modifications have been identified in DNA, including methylation, hydroxymethylation, formylation, and carboxylation. In addition, at least 10 different types of histone modifications have been identified. The key elements involved in these modifications, according to epigenetics, include writers, which include numerous enzymes that can modify the nucleotide bases and specific amino acid residues of histones, and readers, which are a diverse range of proteins with specialized domains that can recognize specific epigenetic marks of a gene allele.

In terms of the writer function, modifications to DNA and histone proteins occur by adding different chemical groups using numerous enzymes. While numerous modifications are possible, the two most widely studied epigenetic modifications, methylation and acetylation, can be the main focus. Both DNA and histone proteins are susceptible to methylation, while acetylation is specific to histones. Both modifications often regulate a cell's gene expression patterns through transcriptional activation or repression. Epigenetic writers include DNA methyltransferases, histone lysine methyltransferases, protein arginine methyltransferases, and histone acetyltransferases.

In terms of the reader function, the numerous modifications made by epigenetic writers must be recognized by other cellular proteins to mediate their effects. In mammalian cells, several protein domains have evolved to bind to these modifications and are known as readers of the epigenome. Numerous chromatin modifiers act as epigenome readers because they have specialized domains that can identify and bind to a variety of covalent modifications present on DNA and histones. Protein domains that can identify methylated DNA can be the main focus. The focus can be on domains that can recognize and bind methylated DNA and two histone modifications: methylation and acetylation.

It is unusual for most histone modifier proteins to perform two functions. Enhancing the reader function may have fewer side effects than enhancing the writer function. Engineering the reader domain holds promise not only for enhancing the effectiveness of cancer therapies, but also for controlling new therapeutic targets.

On the other hand, enhancing the writer function only enhances existing functions and does not provide new therapeutic targets. Taken together, enhancing the reader function can have significant benefits.

Under this technical background, the inventors of the present invention have confirmed a polypeptide comprising a histone code reader domain and use thereof in the prevention or treatment of cancer, and have completed the present invention.

### [Detailed Description of the Invention]

### [Technical Problem]

It is an object of the present invention to provide a polypeptide comprising a histone code reader domain.

It is an object of the present invention to provide a nucleic acid encoding the polypeptide.

It is an object of the present invention to provide an mRNA encoding the polypeptide.

It is an object of the present invention to provide isolated cells producing the nucleic acid.

It is an object of the present invention to provide a delivery vehicle comprising the mRNA.

It is an object of the present invention to provide a composition for the prevention or treatment of cancer comprising the polypeptide, the nucleic acid or the mRNA.

### [Technical solution]

To accomplish the above objectives, the present invention provides a polypeptide comprising:
a polypeptide represented by the structure of D1-D2-D3:
   (a) D1 is the PHD1 domain of PHF6 (PHD Finger Protein 6);
   (b) D2 is the PHD2 domain of PHF6 or variants thereof; and
   (c) D3 includes one or more selected from the group consisting of:
      (i) D2;
      (ii) a fragment of PHF6 containing D2;
      (iii) PHD1 domain of double PHD fingers 3b (DPF3b);
      (iv) PHD2 domain of DPF3b or variants thereof; and
      (v) PHD1 domain and PHD2 domain of DPF3b, or variants thereof.

The present invention provides a nucleic acid encoding the polypeptide.

The present invention provides an mRNA encoding the polypeptide.

The present invention provides an isolated cell producing the nucleic acid.

The present invention provides a delivery vehicle comprising the mRNA.

The present invention provides a composition for preventing or treating cancer comprising the polypeptide, nucleic acid, mRNA, cell or delivery vehicle. The present invention provides a method of preventing or treating cancer comprising the step of administering the polypeptide, nucleic acid, mRNA, cell or delivery vehicle to an individual. The present invention provides the use of the polypeptide, nucleic acid, mRNA, cell or delivery vehicle in the manufacture of an agent for the prevention or treatment of cancer.

### [Brief Description of Drawing]

FIG. 1 is a schematic illustrating the PHF6-based engineering for the creation of chromatin Epi-glue.
FIG. 2 is a schematic representation of the details of a yeast display to improve the binding affinity of the PHF6 reader domain.
FIG. 3 shows the results of testing the binding affinity of ePHD2 for the H2B and H2BK12Ac peptides using a yeast display system.
FIG. 4 shows the results of library construction and library screening for reader domain binding affinity maturation.
FIG. 5 shows the results of analyzing the five ePHD2 variants individually.
FIG. 6 shows an example of creating an Epi-glue protein by replacing the reader domain of PHF6 with another epigenetic reader domain.
FIG. 7 shows the results of analyzing the peak shifts in accordance with H2B peptide concentration titration.
FIG. 8 shows the cell proliferation results in cells expressing ePHD2(T208) after gene transfer using lentivirus.
FIG. 9 shows western blot data from cells expressing ePHD2(T208) after gene transfer using lentivirus.
FIG. 10 shows the results of expressing the synthesized mRNA through in vitro transcription in breast cancer cells and confirming the effect of inhibiting cell proliferation.
FIG. 11 shows the results of identifying increased expression of tumor suppressive miRNAs in the chromatin Epi-glue T208 (DR3) sample.
FIG. 12 shows the results of identifying increased expression of chemokines among cytokines in the chromatin Epi-glue T208 (DR3) sample.
FIG. 13 shows the in vivo efficacy of ePHD2(T208) using an immunodeficient nude mouse model.
FIG. 14 shows the results of validating the efficacy of chromatin Epi-glue in the immunodeficient nude mouse xenograft model.
FIG. 15 shows the expression of genes related to chemokines in the T208 (DR3) sample.
FIG. 16 shows the results of growth inhibition of Epi-glue protein using lymphoblast K562 derived from a CML patient, and promyeloblast HL-60 cell line derived from an AML patient.
FIG. 17 shows the results of growth inhibition of Epi-glue protein using epithelial cancer D283 derived from a medulloblastoma patient, and epithelial cancer HCT116 cell line derived from a colorectal carcinoma patient.
FIG. 18 shows the results of growth inhibition of Epi-glue protein using lymphoblast RPMI6666 derived from a Hodgkin's disease patient, U-87 MG derived from a likely glioblastoma patient, and Daoy cell line derived from a desmoplastic cerebellar medulloblastoma patient.
FIG. 19 shows a schematic of a model designed to test for differences in efficacy based on the length of the linker between two ePHD2 domains (linker).
FIG. 20 shows the results of cell proliferation assays and cell migration inhibition in accordance with the length of the ePHD2 domain linker.

### [Detailed Description of the Invention]

Unless otherwise defined, all technical and scientific terms used herein shall have the same meaning as commonly understood by those skilled in the art. In general, the nomenclature used herein is well known and in common use in the art.

While investigating epigenetic therapeutic agents as candidates for the treatment of cancer, the inventors of the present invention present a polypeptide comprising the PHD1 domain and PHD2 domain of PHF6 (PHD finger protein 6), or variants thereof, that are reversibly modifiable, rather than irreversible, such as by genome editing, and whose therapeutic intensity can be adjusted, for example by introducing variants.

A single histone modifier protein was designed to serve two functions, as a reader and a writer, and the strength of its activity could be regulated by changing each. In particular, enhancing the reader function can increase the safety of the drug and optimize its effectiveness, thereby reducing side effects, which were optimized by modifying the reader domain.

The engineering of reader domains offers new possibilities for controlling therapeutic targets, and the introduction of variation can be made for reversible modulation and control of therapeutic intensity.

Based on this, the present invention relates to a polypeptide comprising a histone code reader domain, specifically a domain of PHF6 (PHD finger protein 6) or variants thereof, and/or a domain of DPF3b (double PHD fingers 3b) or variants thereof, in duplicate, or made by swapping between domains.

More particularly, the present invention relates to a polypeptide represented by the structure of D1-D2-D3:
(a) D1 is the PHD1 domain of PHF6 (PHD finger protein 6);
(b) D2 is the PHD2 domain of PHF6 or variants thereof; and
(c) D3 includes one or more selected from the group consisting of:
   (i) D2;
   (ii) a fragment of PHF6 containing D2;
   (iii) PHD1 domain of double PHD fingers 3b (DPF3b);
   (iv) PHD2 domain of DPF3b or variants thereof; and
   (v) PHD1 domain and PHD2 domain of DPF3b, or variants thereof.

"Domain" can mean any recognizable unit or module that makes up a polypeptide. Some small polypeptides may consist of a single domain, but others may contain multiple domains. Domains. Domains which can be modular building blocks can have structural or functional autonomy and/or independence.

"Swapping" can refer to the interchange of segments between two or more domains. Domains or gene fragments encoding domains can be combined and the resulting polypeptide can be rearranged. While swapping occurs between multiple domains, the remaining domains may retain the same structure except at the points of exchange or modification caused by cleavage of bonds between the domains. This allows for the generation of polypeptides with altered structure or length.

The term "polypeptide" is used interchangeably with "protein," and refers to a polymer of amino acid residues, wherein at least one amino acid residues can be applied to not only naturally or non-naturally occurring amino acid polymers, but also amino acid polymers, which are artificial chemical mimics of the corresponding naturally occurring amino acid.

The term "amino acid" can refer to naturally occurring amino acids, synthetic amino acids, amino acid analogs that function in a similar manner to naturally occurring amino acids, and amino acid mimics. Naturally occurring amino acids include amino acids encoded by the genetic code and amino acids that are later modified (e.g., hydroxyproline, γ-carboxyglutamate, and O-phosphoserine). Amino acid analogs include compounds that have the same basic chemical structure as naturally occurring amino acids, i.e., hydrogen, a carboxyl group, an amino group, and a carbon bonded to an R group, such as homoserine, norleucine, methionine sulfoxide, and methionine methyl sulfonium. These analogs may have a modified R group (e.g., norleucine) or a modified peptide backbone, but retain the same basic chemical structure as the naturally occurring amino acid. Amino acid mimics include compounds that have a structure that differs from the typical chemical structure of an amino acid but function in a similar way to a naturally occurring amino acid. The terms "non-naturally occurring amino acids" and "non-natural amino acids" include amino acid analogs, synthetic amino acids, and amino acid mimics that are not found in nature.

"Histone code readers" are epigenome readers that can play a key role in histone modification crosstalk by recognizing various post-translational modifications (PTMs) and acting as a platform for loading different epigenome regulators. There are multiple domains for reading specific PTMs, including bromodomains for acetylation and chromodomains for methylation.

The domain of the histone code reader is the PHD finger, which can recognize methylated lysines on histones, such as H3K4me2/3, which are mainly associated with transcription start sites of active genes. The PHD finger can play an important role in histone modification crosstalk by reading binding histone PTMs. PHD fingers belong to the zinc-binding superfamily and the tandem PHD domain can detect two zinc ions by conserved cysteine and histidine residues.

PHFs (PHD finger-containing proteins) have diverse functions and structures, and can function as epigenetic factors by interacting with histone modifications through their PHD (plant homeodomain) domains. Among PHFs, PHF6, which has two extended PHDs, can interact with histones to function as a transcriptional regulator, act as a reader of histone modifications, and specifically recognize histone H2BK12 acetylation (H2BK12Ac).

The PHF6 may comprise, for example, a sequence of SEQ ID NO: 1.
[SEQ ID NO: 1]

MSSSIEQKKGSTRQRKCGFCKSNRDKECGQLLISENQKVAAHHKCMLFSSALVSS
HSDNESLGGFSIEDVQKEIKRGTKLMCSLCHCPGATIGCDVKTCHRTYHYHCALHDKAQIRE
KPSQGIYMVYCRKHKKTAHNSEADLEESFNEHELEPSSPKTKKKSRKGRPRKTNLKGLPED
SRSTSSHGTDEMESSSYRDRSPHRSSPNDTRPKCGFCHVGEEENEARGKLHIFNAKKAAA
HYKCMLFSSGTVQLTTTSRAEFGDFDIKTVLQEIKRGKRMKCTLCSQPGATIGCEIKACVKT
YHYHCGVQDKAKYIENMSRGIYKLYCKNHSGNDERDEEDEERESKSRGRVAIDQQLTQQQ
LNGN

The PHD domain of PHF6 (PHD finger protein 6) is a protein domain composed of two β-strands and several short α-helix fragments, which are stabilized by two zinc pincer motifs. Two or more PHD domains can be stacked to recognize and bind specific histone modifications, interact with other histone-binding domains, and function as epigenetic factors.

The present invention provides a polypeptide represented by the structure of D1-D2-D3, wherein:
(a) D1 is the PHD1 domain of PHF6 (PHD finger protein 6);
(b) D2 is the PHD2 domain of PHF6 or variants thereof; and
(c) D3 comprises D2 or a fragment of PHF6 comprising D2.

According to the present invention, a novel polypeptide was designed based on the strategy of repeating the PHD2 (reader) domain of PHF6.

In this case, D1-D2-D3 can have a structure comprising D2 at the C-terminus of D1, and D3 at the C-terminus of D2, from the N-terminus to the C-terminus. Specifically, from N-terminus to the C-terminus, D1-D2-D3 may comprise a PHD1 domain of PHF6-a PHD2 domain of PHF6 or variants thereof-a PHD2 domain of PHF6 or variants thereof, or a fragment of PHF6 comprising the same.

The D1 is the PHD1 domain of PHF6, which may comprise, for example, the sequence of positions 17 to 131 in SEQ ID NO: 1. In particular, D1 may comprise a sequence of SEQ ID NO: 2.
[SEQ ID NO: 2]

CGFCKSNRDKECGQLLISENQKVAAHHKCMLFSSALVSSHSDNESLGGFSIEDVQ
KEIKRGTKLMCSLCHCPGATIGCDVKTCHRTYHYHCALHDKAQIREKPSQGIYMVYCRKH

D2 is the PHD2 domain of PHF6 or variants thereof, wherein the PHD2 domain of D2 may comprise, for example, the sequence of positions 208 to 329 in SEQ ID NO: 1. More particularly, D2 may comprise a sequence of SEQ ID NO: 3.
[SEQ ID NO: 3]

TRPKCGFCHVGEEENEARGKLHIFNAKKAAAHYKCMLFSSGTVQLTTTSRAEFGD
FDIKTVLQEIKRGKRMKCTLCSQPGATIGCEIKACVKTYHYHCGVQDKAKYIENMSRGIYKLY
CKNH

A variant of the PHD2 domain of PHF6 indicates the condition in which one or more amino acids have been substituted and/or modified, resulting in a different amino acid sequence from the unmodified PHD2 domain of PHF6, but retaining its functions or properties. One or more amino acids in the amino acid sequence may be modified, resulting in properties that are increased, unchanged, or decreased compared to those of the unmodified domain.

The substitutions and/or modifications include deletions, insertions, and/or substitutions of amino acid sequence residues. These amino acid modifications are made based on the relative similarity of the amino acid side chain substituents, e.g., hydrophobicity, hydrophilicity, charge, size, etc. By analyzing the size, shape, and type of amino acid side chain substituents, it can be seen that arginine, lysine, and histidine are all positively charged residues; alanine, glycine, and serine have similar sizes; and phenylalanine, tryptophan, and tyrosine have similar shapes. Therefore, based on these considerations, arginine, lysine, and histidine; alanine, glycine, and serine; and phenylalanine, tryptophan, and tyrosine are biologically functional equivalents.

The present invention is construed to include a sequence that exhibits substantial identity to the sequence of the present invention. Substantial identity means a sequence that exhibits at least 90% homology, most preferably at least 95% homology, 96% or more, 97% or more, 98% or more, 99% or more homology, when the sequence of the present invention and any other sequence are aligned to correspond to each other as much as possible and the aligned sequences are analyzed using algorithms known in the art. Alignment methods for sequence comparison are known in the art. A sequence may have 90%, 91%, 92%, 93%, 94%, 95%, 96%, 97%, 98%, 99%, or greater homology to any or all of the specified sequences listed in the specification.

In this case, D1-D2-D3 can have a structure comprising D2 at the C-terminus of D1 and D3 at the C-terminus of D2 from the N-terminus to the C-terminus. Specifically, from the N-terminus to the C-terminus, D1-D2-D3 may comprise a PHD1 domain of PHF6-a PHD2 domain of PHF6-a PHD2 domain of PHF6.

The PHD2 domain variant of PHF6 may comprise one or more of the following amino acid substitutions in SEQ ID NO:1:
C212Y;
G218R;
I230T;
F231S;
N232K;
T253I;
Q270K or Q270R;
C280S;
I290V;
E293G or E293A;
Y301H;
C326R or C326S; and
S346R.

The PHD2 domain variant of PHF6 may comprise any combination of the substitutions specified above in SEQ ID NO: 1. It may include, but is not limited to, combinations of 1, 2, 3, 4, 5, or 6 amino acid substitutions of any of the substitutions specified above.

The PHD2 domain variant of PHF6 may comprise, in particular, the following amino acid substitutions in SEQ ID NO: 1:
V1: T253I, Q270K, E293G, S346R
V2: Q270R, E293A
V3: Q270K, S284R, Y322H, Q359R
V4: C212Y, G218R, I230T, C280S, I290V, C326R; or
V5: F231S, N232K, Y301H, Q270R, C326S.

The PHD2 domain variants of PHF6 may comprise, in particular, V1: T253I, Q270K, E293G, S346R.

In this case, D1-D2-D3 can have a structure from N-terminus to C-terminus comprising D2 at the C-terminus of D1 and D3 at the C-terminus of D2. Specifically, from N-terminus to C-terminus, D1-D2-D3 may comprise a PHD1 domain of PHF6-a PHD2 domain of PHF6 comprising a V2 variant-a PHD2 domain of PHF6 comprising a V2 variant, or a fragment of PHF6 comprising same.

The PHD2 domain variant of PHF6 may comprise, in particular, V2:Q270R, E293A.

In this case, D1-D2-D3 can have a structure from N-terminus to C-terminus comprising D2 at the C-terminus of D1 and D3 at the C-terminus of D2. Specifically, from N-terminus to C-terminus, D1-D2-D3 may comprise a PHD1 domain of PHF6-a PHD2 domain of PHF6 comprising a V2 variant-a PHD2 domain of PHF6 comprising a V2 variant, or a fragment of PHF6 comprising same.

The fragment of PHF6 comprising D2 in D3 may be cleaved before one or more amino acid residues selected from the group consisting of positions 132 to 210 in the sequence of SEQ ID NO: 1. In the fragment of PHF6 comprising D2 in D3, the N-terminus of one or more amino acid residues selected from the group consisting of positions 132 to 210 of the sequence of SEQ ID NO: 1 may be cleaved.

Cleaved before one or more amino acid residues selected from the group consisting of positions 132 to 210 of the sequence of SEQ ID NO: 1 means, for example, cleaved and removed from the amino acid residue at position 1 of the sequence of SEQ ID NO: 1 to the amino acid residue at position 131 immediately preceding the amino acid residue at position 132, based on the N-terminus. For example, it means cleaved from the amino acid residue at position 1 to the amino acid residue at position 209 immediately preceding the amino acid residue at position 210, based on the N-terminus of SEQ ID NO: 1.

A fragment of PHF6 comprising D2 in D3 may be cleaved before an amino acid residue at position 134T or 177E of the sequence of SEQ ID NO: 1.

A cleavage before amino acid residue 134T in the sequence of SEQ ID NO: 1 means, for example, a cleavage from the amino acid residue at position 1 in the sequence of SEQ ID NO: 1 to the amino acid residue at position 133, which is the amino acid residue immediately preceding the 134T amino acid residue, based on the N-terminus of SEQ ID NO: 1. For example, it means cleaved from the amino acid residue at position 1 to the amino acid residue at position 176 immediately preceding the amino acid residue at 177E, based on the N-terminus of SEQ ID NO: 1.

The polypeptide may have a repeat of the reader domain at the C-terminus of PHF6. Since the role of the linker in the function of the reader domain is not known, the following three candidates are provided. The positions of the mutations are labeled relative to the sequence of PHF6, SEQ ID NO:1.

Candidate 1: Repeating the sequence from 134T to ePHD2, including the entire linker connecting ePHD1 and ePHD2.

Candidate 2: Repeating the sequence from 177E to ePHD2 that contain sequences with conserved features in vertebrate species among the linker sequences.

Candidate 3: Excluding the linker sequence and repeating only the reader domain (from 208T).

Variants found by binding affinity maturation can be applied:
Candidate 4: Replacing the wild type reader domain with variant 1 (T253I, Q270K, E293G, S346R).
Candidate 5: Replacing the wild type reader domain with variant 2 (Q270R, E293A).
Candidate 6: Replacing the reader domain with variant 1 in the #7 construct, which resulted in increased ubiquitination efficiency.

Specifically, the polypeptide may comprise the sequence of SEQ ID NO: 6:

MSSSIEQKKGSTRQRKCGFCKSNRDKECGQLLISENQKVAAHHKCMLFSSALVSS
HSDNESLGGFSIEDVQKEIKRGTKLMCSLCHCPGATIGCDVKTCHRTYHYHCALHDKAQIRE

KPSQGIYMVYCRKHKKTAHNSEADLEESFNEHELEPSSPKTKKKSRKGRPRKTNLKGLPED
SRSTSSHGTDEMESSSYRDRSPHRSSPNDTRPKCGFCHVGEEENEARGKLHIFNAKKAAA
HYKCMLFSSGTVQLTTTSRAEFGDFDIKTVLQEIKRGKRMKCTLCSQPGATIGCEIKACVKT
YHYHCGVQDKAKYIENMSRGIYKLYCKNHSGNDERDEEDEERESKSRGRVAIDQQLTQQQ
LNGNHMGSGGGGPGTRPKCGFCHVGEEENEARGKLHIFNAKKAAAHYKCMLFSSGTVQL
TTTSRAEFGDFDIKTVLQEIKRGKRMKCTLCSQPGATIGCEIKACVKTYHYHCGVQDKAKYI
ENMSRGIYKLYCKNHSGNDERDEEDEERESKSRGRVAIDQQLTQQQLNGN

The present invention has also screened for novel domains that are swappable with the existing H2BK12Ac reading ability. Based on this, the present invention comprises a polypeptide represented by the structure D1-D2-D3, wherein:
(a) D1 is the PHD1 domain of PHF6 (PHD finger protein 6);
(b) D2 is the PHD2 domain of PHF6 or variants thereof; and
(c) D3 comprises at least one selected from the group consisting of:
   (iii) PHD1 domain of double PHD fingers 3b (DPF3b);
   (iv) PHD2 domain of DPF3b or variants thereof; and
   (v) PHD1 domain and PHD2 domain of DPF3b or variants thereof.

The description on D1 and D2 may be applied as above. D3 comprises a PHD1 domain and a PHD2 domain of DPF3b (Double PHD fingers 3b) or variants thereof, or a DPF3b fragment comprising a PHD1 or PHD2 domain or variants thereof.

The PHD (plant homeodomain) finger of the DPF3b can function as a reader of acetylated histones. The DPF3b is a member of the d4 protein family and consists of an N-terminal 2/3 domain, a C2H2-Kruppel-like zinc finger and a C-terminal tandem PHD zinc finger. The DPF3b interacts with histones H3 and H4 at lysine, and does so in an acetylation-dependent manner. The tandem PHD fingers of DPF3b bind to the N-terminal tail of histone H3, and acetylation of the H3 lysine 14 site (H3K14ac) enhances the interaction, whereas methylation of the lysine 4 site (H3K4me) inhibits binding.

The first PHD finger (PHD1) domain of DPF3b binds to H3K14ac, while the second PHD finger (PHD2) domain binds to the H3R2-K4 region. DPF3b-PHD2 is similar to the H3K4me0-binding PHD fingers (BHC80, AIRE, DNMT3L), sharing a combination of acidic and hydrophobic residues in the H3K4me0 binding pocket. However, the H3K14ac binding pocket of DPF3b-PHD1 is located on the opposite side of the central β-sheet and utilizes a set of residues that do not overlap with those used to form the H3K4 or H3R2 binding sites. Nevertheless, the H3K14ac binding site follows the same principle of using a structurally tight position near a Zn-coordinating Cys residue or β2-strand (Trends Biochem Sci. 2011 Jul; 36(7): 364-372; The PHD Finger: A Versatile Epigenome Reader).

The PHD1 domain of the DPF3b may comprise, for example, a sequence of SEQ ID NO: 4. The PHD2 domain of DPF3b may comprise, for example, a sequence of SEQ ID NO: 5.
[SEQ ID NO: 4] 
YCDFCLGGSNMNKKSGRPEELVSCADCGRSGHPTCLQFTLNMTEAVKTYKWQCI
EC
[SEQ ID NO: 5]

SCILCGTSENDDQLLFCDDCDRGYHMYCLNPPVAEPPEGSWSCHLCWELLKEKA

SAFGCQA

A variant of the PHD2 domain of DPF3b indicates the condition in which one or more amino acids have been substituted and/or modified such that the amino acid sequence differs from that of the unmodified PHD2 domain of DPF3b, but functions or properties are maintained. One or more amino acids in the amino acid sequence may be modified, resulting in properties that are increased, unchanged, or decreased compared to those of the unmodified domain.

The substitutions and/or modifications include deletions, insertions, and/or substitutions of amino acid sequence residues. These amino acid modifications are made based on the relative similarity of the amino acid side chain substituents, e.g., hydrophobicity, hydrophilicity, charge, size, etc. By analyzing the size, shape, and type of amino acid side chain substituents, it can be seen that arginine, lysine, and histidine are all positively charged residues; alanine, glycine, and serine have similar sizes; and phenylalanine, tryptophan, and tyrosine have similar shapes. Therefore, based on these considerations, arginine, lysine, and histidine; alanine, glycine, and serine; and phenylalanine, tryptophan, and tyrosine are biologically functional equivalents.

The present invention is construed to include a sequence that exhibits substantial identity to the sequence of the present invention. Substantial identity means a sequence that exhibits at least 90% homology, most preferably at least 95% homology, 96% or more, 97% or more, 98% or more, 99% or more homology, when the sequence of the present invention and any other sequence are aligned to correspond to each other as much as possible and the aligned sequences are analyzed using algorithms known in the art. Alignment methods for sequence comparison are known in the art. A sequence may have 90%, 91%, 92%, 93%, 94%, 95%, 96%, 97%, 98%, 99%, or greater homology to any or all of the specified sequences listed in the specification.

According to a specific example of the present invention, in a group of candidates for a polypeptide comprising the PHD1 domain of PHF6 repeating the PHD2 (reader) domain of PHF6 - ePHD2(T208)-ePHD2(T208), and a polypeptide comprising the PHD1 domain of PHF6 with the reader domain swapped - PHF6 ePHD2 - the PHD1 and/or PHD2 domains of DPF3b (hereinafter, Epi-glue), it was found that the writer function, H2BK120Ubrk, was increased, showing that enhanced function of the reader domain can lead to enhanced function of the writer.

The present invention relates to a nucleic acid encoding the polypeptides. "Nucleic acid" means a nucleic acid molecule that is isolated from at least about 50% of the proteins, lipids, carbohydrates, or other substances found in nature together with it when the entire nucleic acid is isolated from the source cell, or is operably linked to a polynucleotide that is not naturally linked, or is not naturally occurring as part of a larger polynucleotide sequence. Specifically, the isolated nucleic acid molecule is substantially free of any other contaminating nucleic acid molecule, or any other contaminant found in its natural environment that would interfere with the production of the polypeptide or its therapeutic, diagnostic, preventive, or research use.

The terms "polynucleotide," "nucleic acid," "nucleic acid molecule," "nucleic acid oligomer," "oligonucleotide," "nucleic acid sequence," and "nucleic acid fragment" are used interchangeably and may include, but are not limited to, nucleotides, deoxyribonucleotides, or ribonucleotides, or analogs, derivatives, or modifications thereof, in the form of polymers covalently linked to each other that may be of varying lengths.

Different polynucleotides can have different three-dimensional structures and can perform a variety of known and unknown functions. Polynucleotides may include, for example, but are not limited to, genes, gene fragments, exons, introns, intergenic DNA (including exotic DNA, but not limited thereto), messenger RNA (mRNA), transfer RNA, ribosomal RNA, ribozymes, cDNA, recombinant polynucleotides, branched polynucleotides, plasmids, vectors, sequenced isolated DNA, sequenced isolated RNA, nucleic acid probes, and primers.

The polynucleotide can typically contain four nucleotide bases: adenine (A), cytosine (C), guanine (G), and thymine (T) (denoted as uracil (U) for thymine (T) if the polynucleotide is RNA).

A "sequence" is an alphabetical representation of a molecule, which can be entered into a database on a computer with a central processing unit and used in bioinformatics applications such as functional genomics and homology searches. A polynucleotide may optionally include one or more non-standard nucleotides, nucleotide analogs, and/or modified nucleotides.

"Nucleic acid" may include deoxyribonucleotides or ribonucleotides in single, double, or multi-stranded form, and polymers thereof or complements thereof. A "polynucleotide" may include a linear sequence of nucleotides.

"Nucleotide" generally refers to a single unit of polynucleotide. Nucleotides can be ribonucleotides, deoxyribonucleotides, or modified forms thereof. Examples of polynucleotides can include single- and double-stranded DNA, single- and double-stranded RNA (including siRNA), and hybrid molecules having mixtures of single- and double-stranded DNA and RNA.

The nucleic acids comprehensively include DNA (gDNA and cDNA) and RNA molecules, and may be mRNA.

Nucleotides, the basic building blocks of nucleic acids, include not only naturally occurring nucleotides but also analogs with modified sugar or base site. The sequences of the nucleic acids encoding the heavy and light chain variable regions of the present invention may be modified. The modifications include additions, deletions, or non-conservative or conservative substitutions of nucleotides.

The nucleic acid may be in the form of ribonucleic acid, for example messenger ribonucleic acid mRNA. The nucleic acid according to the present invention may be in the form of mRNA. This allows for transient protein expression.

The expression level of a DNA molecule in a cell can be determined based on the amount of the corresponding mRNA present in the cell or the amount of protein encoded by the corresponding DNA produced by the cell.

The composition may comprise a delivery vehicle for delivering the mRNA expressed in the composition.

The mRNA may be delivered, for example, via a vector. The vector may be a viral vector. The viral vector may be a retrovirus, an adenovirus parvovirus (e.g., adenoassociated virus (AAV)), a coronavirus, negative-strand RNA viruses such as an orthomyxovirus (e.g., influenza virus), a rhabdovirus (e.g., rabies and vesicular stomatitis virus), a paramyxovirus (e.g., positive-stranded RNA viruses such as measles and Sendai, alphavirus, and picornavirus), a herpesvirus (e.g., Herpes Simplex virus types 1 and 2, Epstein-Barr virus, cytomegalovirus), double-stranded DNA viruses including adenoviruses, or poxviruses (e.g., vaccinia, fowlpox, or canarypox).

The vectors can be delivered in vivo or intracellularly via electroporation, lipofection, viral vectors, nanoparticles, as well as PTD (protein translocation domain) fusion protein methods, respectively.

The expressed mRNA can be delivered, for example, via nanoparticles.

The expressed mRNA can be delivered via liposomes, lipid nano-particles (LNPs) or various nanoparticles. The liposomes or LNPs may comprise cationic lipids, non-cationic lipids, or neutral lipids, and may additionally comprise other lipids such as polyethylene glycol (PEG) or cholesterol. Such mRNA delivery vehicles are described in detail in U.S. Patent Application Publication Nos. 2018/0311176, 2019/0032051, and 2021/0046192, and International Patent Publication Nos. WO2018/081480, WO2020/097540, WO2020/097548, and WO2021/007278, which are incorporated herein by reference.

PEG fats can be included to prevent aggregation of the particles generated upon delivery of the mRNA. PEG fats are specifically described in U.S. Patent Application Publication Nos. 2018/0311176, 2019/0032051, etc., and include, for example, PEG-diacylglycerol (DAG), a PEG-dialkyloxypropyl (DAA), a PEG-phospholipid, a PEG-ceramide (Cer), or a mixture thereof. As a non-limiting example, PLGA may be conjugated to a lipid-terminating PEG forming PLGA-DSPE-PEG, PEG lipid is selected from PEG-c-DOMG and 1,2-Dimyristoyl-sn-glycerol, methoxypolyethylene Glycol (PEG-DMG), 1,2-Distearoyl-sn-glycerol, methoxypolyethylene Glycol (PEG-DSG), PEG-c-DOMG, 1,2-Distearoyl-sn-glycerol, methoxypolyethylene glycol (PEG-DSG) 1,2-Dipalmitoyl-sn-glycerol, methoxypolyethylene glycol (PEG-DPG), PEG-lipid conjugates such as, e.g., PEG coupled to dialkyloxypropyls (e.g., PEG-DAA conjugates), PEG coupled to diacylglycerols (e.g., PEG-DAG conjugates), PEG coupled to cholesterol, PEG coupled to phosphatidylethanolamines, and PEG conjugated to ceramides, cationic PEG lipids, polyoxazoline (POZ)-lipid conjugates, polyamide oligomers (e.g., ATTA-lipid conjugates), and mixtures thereof. In some examples, the PEG is a PEG-dilauryloxypropyl (C12), a PEG-dimyristyloxypropyl (C14), a PEG-dipalmityloxypropyl (C16), a PEG-distearyloxypropyl (C18), PEG-c-DOMG, PEG-DMG, or mixtures thereof, but is not limited thereto.

Peptides may be used for the mRNA delivery. The peptide must have a cation to electrostatically interact with the anionic phosphate group of the nucleic acid, and may include a positively charged amino acid to electrostatically interact with the phosphate group.

Cell-permeable peptides (CPPs) may also be promising cationic molecules for the delivery of mRNA.

In some cases, in addition to the liposome or lipid nano-particle (LNP), the mRNA delivery may further include a peptide. The peptide may provide nucleic acid packaging and protect the DNA or RNA from intracellular or extracellular degradation.

The composition may further comprise one or more pharmaceutically acceptable carriers. The pharmaceutically acceptable carrier must be compatible with the active ingredient of the present invention and can be saline, sterile water, Ringer's solution, buffered saline, dextrose solution, maltodextrin solution, glycerol, ethanol, and mixtures of one or more of these components, and other conventional additives such as antioxidants, buffers, bacteriostatic agents, etc. can be added as needed. Furthermore, diluents, dispersants, surfactants, binders, and lubricants can be further added to formulate them into injectable formulations such as aqueous solutions, suspensions, emulsions, and the like. In particular, it is preferable to formulate and provide the formulation in lyophilized form. For the preparation of lyophilized formulations, methods known in the art may be used, and stabilizing agents for lyophilization may be added. Furthermore, the components can be preferably formulated according to the respective disease or the ingredients by any suitable method in the art or by using the method disclosed in Remington's pharmaceutical Science, Mack Publishing company, Easton PA.

The content of the active ingredient in the composition of the present invention and the method of administration can be determined by a person of ordinary skill in the art based on the symptoms of the patient and the severity of the disease. They can also be formulated in various forms, such as pills, tablets, capsules, liquids, injections, ointments, syrups, and the like, and can be provided in unit-dose or multi-dose containers, such as sealed ampoules and bottles.

The composition of the present invention can be administered orally or parenterally. Routes of administration of the composition according to the present invention include, but are not limited to, bronchial, oral, intravenous, intramuscular, intra-arterial, intramedullary, intrathecal, intracardiac, transdermal, subcutaneous, intraperitoneal, enteral, sublingual, or topical administration. The dosage of the composition according to the present invention may vary depending on the patient's weight, age, gender, health status, diet, time, method, excretion rate, or severity of the disease, and can be readily determined by a person of ordinary skill in the art. Furthermore, the composition of the present invention can be formulated into a suitable dosage form for clinical administration using the known techniques.

DNA is easily isolated or synthesized using conventional procedures. Many vectors are available. Vector components typically include, but are not limited to, one or more of the following: a signal sequence, a replication site, one or more marker genes, an enhancer element, a promoter, and a transcription termination sequence.

As used herein, the term "vector" includes plasmid vectors; cosmid vectors; and viral vectors, such as bacteriophage vectors, adenoviral vectors, retroviral vectors, and adenoassociated viral vectors, as a means for expressing a target gene in a host cell.

"Operationally linked" means a functional association between a nucleic acid expression regulatory sequence (e.g., a promoter, signal sequence, or array of transcriptional regulator binding sites) and another nucleic acid sequence, whereby the regulatory sequence regulates the transcription and/or decoding of the other nucleic acid sequence.

In the case of prokaryotic cells as hosts, it is common to include a strong promoter capable of driving transcription (e.g., a tac promoter, lac promoter, lacUV5 promoter, Ipp promoter, pLλ promoter, pRλ promoter, rac5 promoter, amp promoter, recA promoter, SP6 promoter, trp promoter, and T7 promoter), a rhizosome binding site for initiation of decoding, and a transcription/degradation termination sequence. In addition, for example, in the case of eukaryotic cells as hosts, the promoter may comprise a promoter derived from the genome of a mammalian cell (e.g., metallothionein promoter, β-actin promoter, human hemoglobin promoter, and human muscle creatine promoter) or a promoter derived from a mammalian virus (e.g., adenovirus late promoter, vaccinia virus 7.5K promoter, vaccinia virus 7.5K promoter, SV40 promoter, cytomegalovirus (CMV) promoter, tk promoter of HSV, mouse mammary tumor virus (MMTV) promoter, LTR promoter of HIV, Moloney virus promoter, Epstein-Barr virus (EBV) promoter, and Roos Sakoma virus (RSV) promoter) may be utilized and typically have a polyadenylation sequence as a transcription termination sequence.

The cell is an isolated cell comprising the nucleic acid. It can use Escherichia coli, strains of the genus Bacillus, such as Bacillus subtilis and Bacillus churrigensis, Streptomyces, Pseudomonas (e.g, Pseudomonas putida), Proteus mirabilis, and prokaryotic host cells such as Staphylococcus (e.g., Staphylococcus carnosus).

Examples of useful host cell lines include, but are not limited to, COS-7, BHK, CHO, CHOK1, DXB-11, DG-44, CHO/-DHFR, CV1, COS-7, HEK293, BHK, TM4, VERO, HELA, MDCK, BRL 3A, W138, Hep G2, SK-Hep, MMT, TRI, MRC 5, FS4, 3T3, RIN, A549, PC12, K562, PER.C6, SP2/0, NS-0, U20S, and HT1080.

The cells may comprise, for example, immune cells for treatment. The nucleic acid may be introduced into an immune cell. The cells may be selected from the group consisting of, for example, but are not limited to, T cells, NK cells, cytokine induced killer cells (CIKs), activated cytotoxic T lymphocytes (CTLs), macrophages, tumor-infiltrating lymphocytes (TILs), and dendritic cells.

The present invention is a composition for the prevention or treatment of cancer comprising the polypeptide, nucleic acid, mRNA, cell or delivery vehicle. The present invention provides a method of preventing or treating cancer comprising the step of administering the polypeptide, nucleic acid, mRNA, cell or delivery vehicle to an individual. The present invention provides use of the polypeptide, nucleic acid, mRNA, cell or delivery vehicle in the prevention of cancer or the manufacture of a therapeutic agent for cancer.

The cancer may be a solid tumor, for example, a breast cancer. According to the present invention, mRNA synthesized by in vitro transcription was used to express in breast cancer cells. Under the conditions of expressing a polypeptide comprising the PHD1 domain of PHF6 - ePHD2(T208) - ePHD2(T208) (Chromatin Epi-glue T208), the effect of inhibiting cell proliferation was confirmed. Since it showed a significant reduction compared to other molecules that either promoted proliferation or had no effect, a method to deliver it to the cells was devised. This provided evidence that chromatin Epi-glue T208 could be used as a therapeutic agent in breast cancer.

Furthermore, the cancer may be, for example, a hematologic cancer, medulloblastoma, colon cancer, or head and neck cancer. According to the present invention, mRNA synthesized by in vitro transcription was expressed in breast cancer cells. Several candidate compounds according to the present invention have been tested for their ability to inhibit cell proliferation.

The pharmaceutically acceptable carriers included in the composition of the invention are those customarily used in pharmaceutical preparations, such as, but are not limited to, lactose, dextrose, sucrose, sorbitol, mannitol, starch, acacia gum, calcium phosphate, alginate, gelatin, calcium silicate, microcrystalline cellulose, polyvinylpyrrolidone, water, syrup, methyl cellulose, methylhydroxybenzoate, propylhydroxybenzoate, talc, magnesium stearate, or mineral oil. In addition to the above components, the composition of the present invention may further comprise lubricants, wetting agents, sweeteners, flavoring agents, emulsifiers, suspending agents, preservatives, and the like.

The pharmaceutical composition of the present invention can be administered orally or parenterally and, if parenterally administered, can be administered by intravenous infusion, subcutaneous infusion, intramuscular infusion, intraperitoneal infusion, endothelial administration, topical administration, intranasal administration, intrapulmonary administration, and rectal administration.

Because proteins or peptides are digestible when administered orally, oral compositions should be formulated to coat the active agent or to protect it from degradation in the stomach. Furthermore, the pharmaceutical composition can be administered by any device that allows the active substance to be transported to the target cells.

Suitable dosages of the composition according to the present invention vary depending on factors such as method of formulation, mode of administration, patient age, weight, sex, pathological conditions, food, time of administration, route of administration, rate of excretion, and response sensitivity, and the ordinarily skilled physician can readily determine and prescribe a dosage effective for the desired treatment or prevention. For example, the daily dosage of a pharmaceutical composition of the present invention is 0.0001-100 mg/kg. As used herein, the term "pharmaceutically effective amount" means an amount sufficient to prevent or treat a cancer or autoimmune disease.

The pharmaceutical composition of the present invention may be prepared in unit dose form or by formulation with pharmaceutically acceptable carriers and/or excipients in accordance with methods readily practiced by a person of ordinary skill in the art. The formulation may be in the form of a solution, suspension, or emulsion in an oil or aqueous medium, or in the form of an excipient, acid, suspension, powder, granule, tablet, or capsule, and may further comprise a dispersant or stabilizing agent.

The pharmaceutical composition of the present invention may be administered orally or parenterally, and when provided parentally, such as intravenously, subcutaneously, ophthalmically, intraperitoneally, intramuscularly, orally, rectally, intraorbitally, intracerebrally, intracranially, intraspinally, intraventricularly, intrathecally, intracistenally, intracapsularly, or intranasally or via aerosol administration, the pharmaceutical composition may comprise, for example, a portion of an aqueous or physiologically applicable body fluid suspension or solution. Accordingly, a carrier or vehicle is physiologically acceptable and can be added to the composition and delivered to the patient. Thus, a carrier such as a body fluid for a formulation may typically include normal saline.

Because proteins or peptides are digestible when administered orally, oral composition should be formulated to coat the active agent or to protect it from degradation in the stomach. Furthermore, the pharmaceutical composition can be administered by any device that allows the active substance to be transported to the target cells.

The frequency of administration will depend on pharmacokinetic parameters. Typically, the clinician will administer the pharmaceutical composition until a dose achieves the intended effect. Thus, the pharmaceutical composition may be administered as a single dose, as two or more time-spaced doses, or as a continuous infusion via an implanted device or catheter. Further refinement of the appropriate dosage is routinely made by those skilled in the art and falls within the scope of work routinely performed by them.

Suitable dosages of the composition according to the present invention vary depending on factors such as method of formulation, way of administration, patient age, weight, sex, pathological conditions, food, time of administration, route of administration, rate of excretion, and response sensitivity, and the ordinarily skilled physician can readily determine and prescribe a dosage effective for the desired treatment or prevention. For example, the daily dosage of a pharmaceutical composition of the present invention is 0.0001-100 mg/kg.

The present invention will now be described in more detail with reference to the following examples. These examples are intended solely to illustrate the present invention, and it will be apparent to one of ordinary skill in the art that the scope of the present invention is not to be construed as limited by these examples.

Example 1: Engineering based on PHF6 to create chromatin Epi-glue

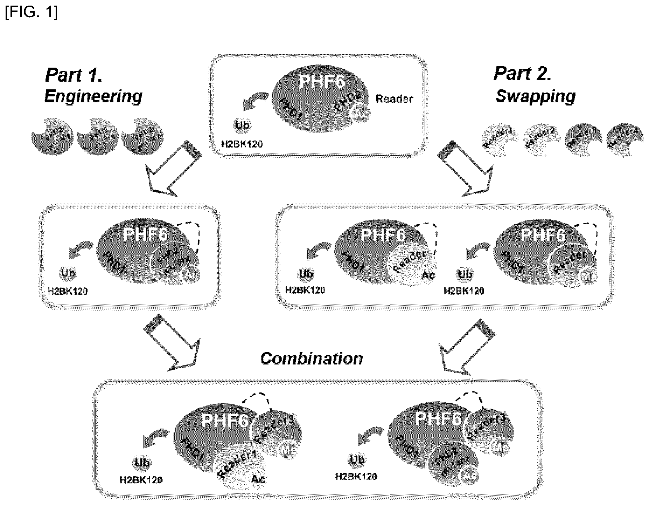

The PHF6-based engineering for the creation of chromatin Epi-glue was as follows (FIG. 1):

### 1. Engineering

### (1) Binding affinity engineering

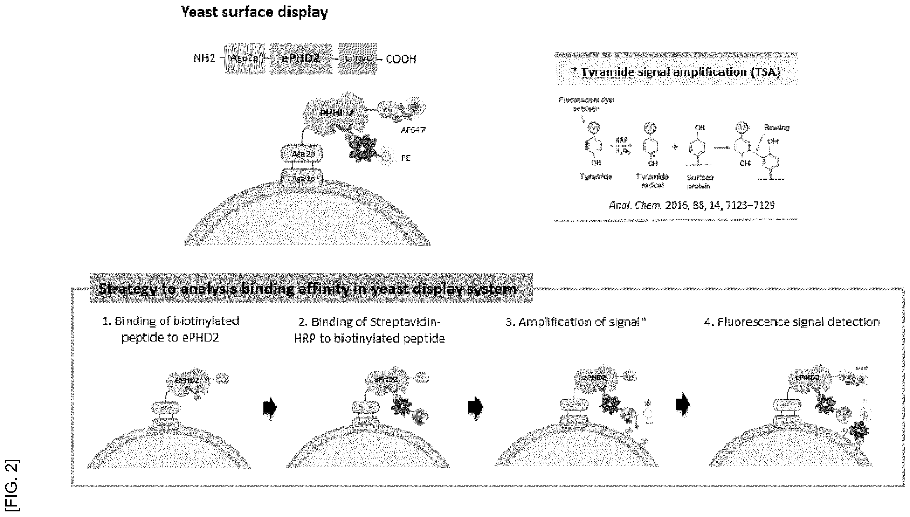

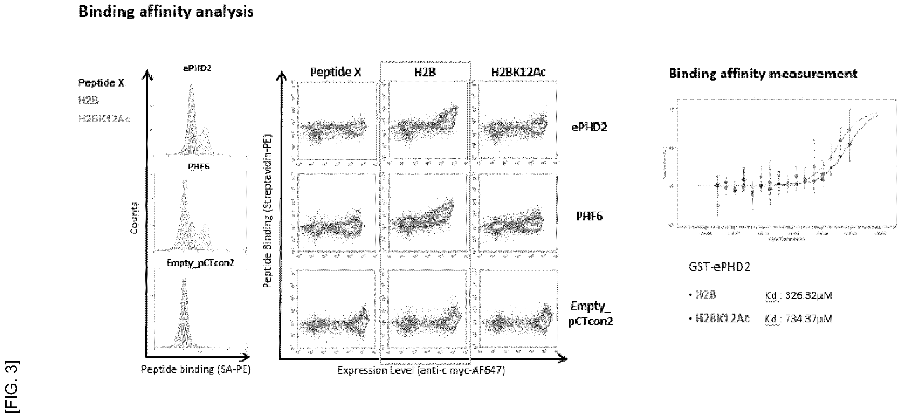

The binding affinity of the PHF6 reader domain was improved. Yeast display technology was used. The details of the yeast display are described in FIG. 2. The binding affinity comparison validity of the yeast display system was evaluated. The binding affinity of the yeast display system was tested for the H2B and H2BK12Ac peptides of ePHD2 containing the sequence of SEQ ID NO: 3. According to the left figure in FIG. 3, a significantly increased binding signal peak was observed in yeast treated with the H2B peptide. The same trend was observed in yeast expressing ePHD2 and PHF6 containing the sequence of SEQ ID NO: 1. The co-vector (empty_pCTcon2, an empty plasmid with no protein sequence) showed almost no binding signal.

Microscale Thermophoresis (MST) was used to exploit the difference in particle motility along a temperature gradient between the ligand bound complex and unbound protein states of a protein.

Proteins were labeled with fluorescent molecules to distinguish the binding of the protein to the ligand by the difference in fluorescence signal for each sample of ligand concentration. The binding affinity (Kd) was then analyzed.

The middle figure in FIG. 3 is a dot plot showing that the peptide binding signal of the expression population in yeast expressing ePHD2 and PHF6 was significantly increased when treated with H2B peptide (see orange box). Therefore, it was determined that the reader domain of PHF6 specifically recognizes H2B peptide.

The right figure in FIG. 3 shows the results of comparing the binding affinity of H2B and H2BK12Ac peptides by MST after the reader domain (ePHD2) was expressed and purified in E.coli. Consistent with the yeast display results, the H2B peptide showed higher binding affinity than the H2BK12Ac peptide.

### (2) Building and screening libraries

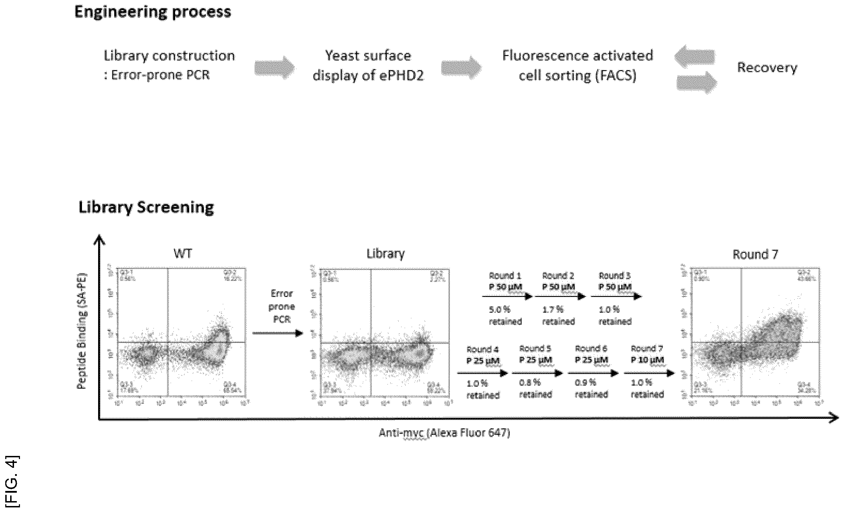

For reader domain binding affinity maturation, the library was constructed as shown in FIG.4, labeled the yeast library using the strategy described in (1) with the addition of TSA, and then screened by fluorescence activated cell sorting (FACS), a flow cytometry technique that detects fluorescence signals by shedding fluorescently labeled cells one by one and sorting only cells with the desired fluorescence intensity. The process was repeated with a recovery step.

The library was subjected to random mutagenesis by error-prone PCR, as the residues involved in binding to the H2B peptide were unknown. The peptide binding signal increased and the FACS process was repeated until saturation.

A total of seven FACS' were performed, decreasing the concentration of the peptide from 50 µM to 10 µM according to the flowchart at the top of FIG. 4.

It can be seen that a population with a higher peptide binding signal was formed in round 7 (bottom right dot plot in FIG. 4) in which FACS was repeated more than in the initial library (bottom center dot plot in FIG. 4). At round 7, the peptide binding signal was no longer increasing, so the FACS was not repeated any further.

As a result of sequencing of round 7, five enriched variants were found.

### (3) Analysis of five ePHD2 variants individually

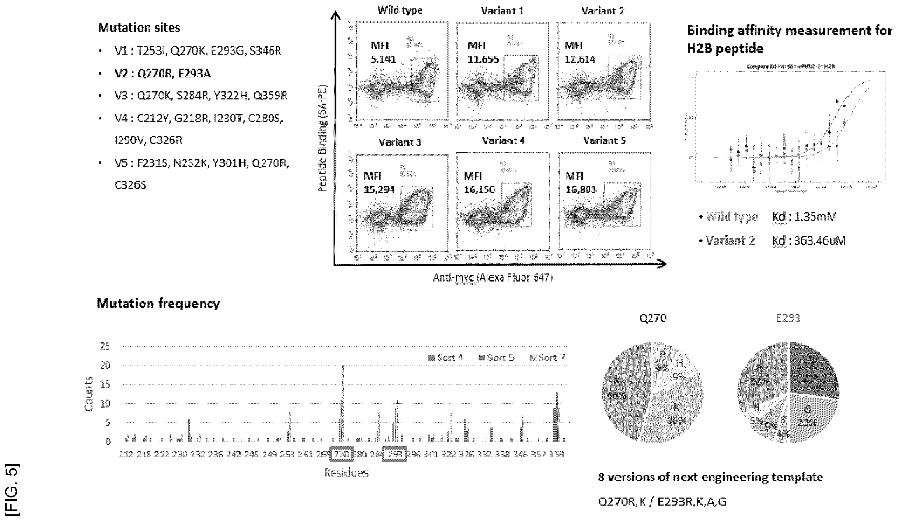

The ePHD2 variant containing the sequence of SEQ ID NO: 3, the positions and amino acids of the mutations in the five variants found in Round 7 are shown below (FIG. 5, top left). The positions of the mutations are labeled relative to SEQ ID NO: 1, which is the sequence of PHF6.
V1: T253I, Q270K, E293G, S346R
V2: Q270R, E293A
V3: Q270K, S284R, Y322H, Q359R
V4: C212Y, G218R, I230T, C280S, I290V, C326R; or
V5: F231S, N232K, Y301H, Q270R, C326S.

According to the top center of FIG. 5, each variant was expressed in yeast to determine if it exhibited a peptide binding signal in the same system. All five variants showed a higher peptide binding signal than the wild type.

According to the top right of FIG. 5, variants 1, 2, and 3 were expressed and purified in E.coli and their binding affinities were measured by MST. Variant 2 showed a lower Kd value for the H2B peptide than the wild type. It is concluded that variant 2 has an enhanced binding affinity.

Variants 4 and 5 were excluded because they have a mutation in a cysteine involved in zinc ion chelation and are not considered to maintain the original structure of ePHD2. ePHD2 has a zinc finger domain, where three zinc ions are chelated and play a role in folding.

Mutation preference was analyzed by sequencing sorts 4, 5, and 7 of the 1^{st} library, as shown in the bottom left of FIG.5. There were common frequent mutation sites. The 1^{st} library is the library generated by the error-prone PCR mentioned in (1), with a library size of approximately 1×10⁷.

It was observed that the mutation frequency at positions 270 and 293 increased with each iteration of FACS.

As shown in the lower right corner of FIG. 5, 2^{nd} library was created based on the mutation frequency information obtained from 1^{st} library. It was intended to find out the mutation preference of a particular site.

2^{nd} library selected positions 270, 293, 326, 334, and 359 that showed high mutation frequency in 1^{st} library sort 4. Sort 4 indicates a library where the peptide binding signal is starting to be higher than that of the wild type.

A site saturation mutagenesis library was constructed with NNK codons as primers at 5 positions. After one round of MACS and three rounds of FACS, the sequencing results were analyzed.

Amino acid preferences of specific properties were observed in Q270 and E293. Q270 had a high proportion of R and K with positive charges. E293 had a high proportion of cationic R, and A and G with small, hydrophobic side chains.

### 2. Swapping

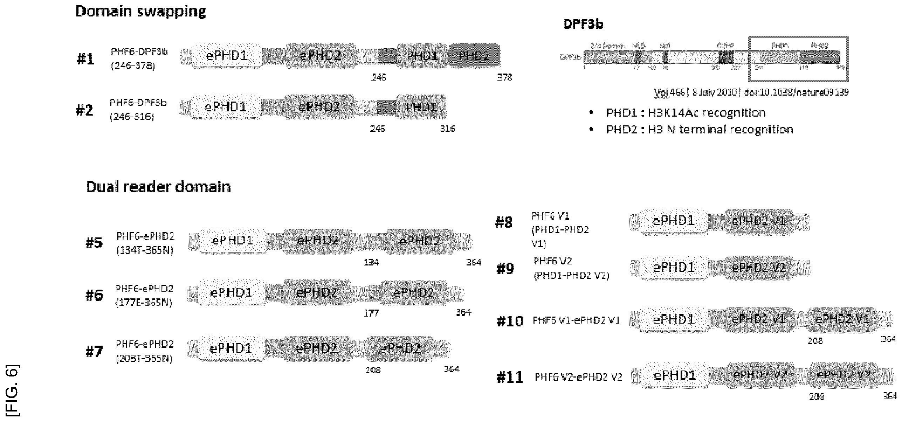

The reader domain of PHF6 was replaced with an engineered reader domain to create the Epi-glue protein. The inventors searched for other epigenetic reader domains that have been reported to recognize epigenetic histone marks. The reader domain of PHF6 was replaced with another epigenetic reader domain to create an Epi-glue protein. A specific example of this is shown in FIG. 6.

### (1) Domain swapping

#1: Linking the PHD1-PHD2 domain of DPF3b, which is known to recognize H3K14Ac, to the C term of PHF6.
#2: PHD1 and PHD2 of DPF3b were found to be responsible for H3K14Ac binding and H3N-terminal binding, respectively (see FIG. 6, top right, first figure). Therefore, only PHD2, which is more directly involved in H3K14Ac binding, can associate with PHF6.

### (2) Dual reader domain

The avidity effect was intended by repeating the reader domain at the C-terminus of PHF6. Since the role of the linker in the function of the reader domain was not known, the following three candidates were created. Mutation positions are labeled relative to the sequence of PHF6, SEQ ID NO: 1.
#5: Repeating the sequence from 134T to ePHD2, including the entire linker connecting ePHD1 and ePHD2.
#6: Repeating the sequence from 177E to ePHD2 including the sequence with conserved features in vertebrate species among the linker sequences.
#7: Repeating only the reader domain, excluding the linker sequence (from 208T).

Variants found by binding affinity maturation can be applied:
#8: Replacing the wild type reader domain with variant 1 (T253I, Q270K, E293G, S346R).
#9: Replacing the wild type reader domain with variant 2 (Q270R, E293A).
#10: Replacing the reader domain with variant 1 in the #7 construct, which resulted in increased ubiquitination efficiency.
#11: Replacing the reader domain with variant 2 in the #7 construct, which resulted in increased ubiquitination efficiency.

### Example 2. Binding structure analysis (NMR) of reader domain and H2B Peptide

The binding structure of the reader domain to the H2B peptide was analyzed by¹H-¹⁵N HSQC. The amide bond of the protein was measured by isotopically labeling it. One peak represents the amide group of one residue. Therefore, the peak position provides information about the protein structure. The shift of the peak upon ligand titration reveals the residues involved in the interaction with the ligand.

The peak shift according to the H2B peptide concentration titration was analyzed. Residues involved in H2B peptide binding were identified.

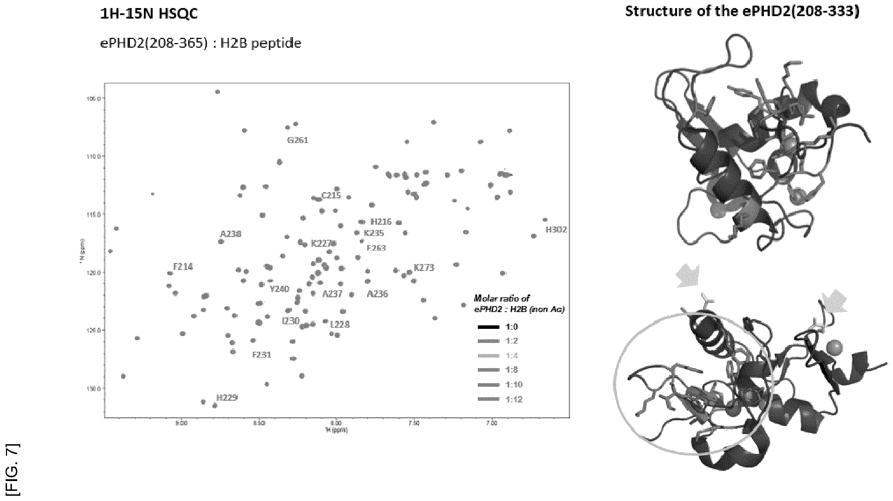

The left side of FIG. 7 shows ¹H-¹⁵N HSQC. Residues colored in red = assigned residues showing peak shifts (using ones assigned from doi: 10.1074/jbc.M113.535351). On the right side of FIG. 7, residues with peak shifts in the ePHD2(208-333) structure (doi: 10.1074/jbc.M113.535351) are colored in red, and the mutation site of variant 2 is colored in light green.

### Example 3. Affinity-enhanced Epi-glue screening

A new Epi-glue was designed by repeating a reader domain. A reader domain repeat Epi-glue with varying linker lengths between PHD1 and PHD2 was designed.
134T-364N: Including the entire linker connecting ePHD1 and ePHD2.
177E-364N: Containing sequences conserved in vertebrate species among linkers.
208T-364N: Containing no linker.

The reader domain of DPF3b, which recognizes H3K14Ac, was used. According to the PHD1: H3K14Ac binding and PHD2: H3 N term binding of DPF3b, it is as follows:
Candidate 1. PHF6-DPF3b (246-378): Using both reader domains PHD1-PHD2 of DPF3b.
Candidate 2. PHF6-DPF3b (246-316): Using only PHD2, which is directly involved in H3K14Ac binding.

### Example 4. Identifying chromatin Epi-glue candidates for breast cancer treatment

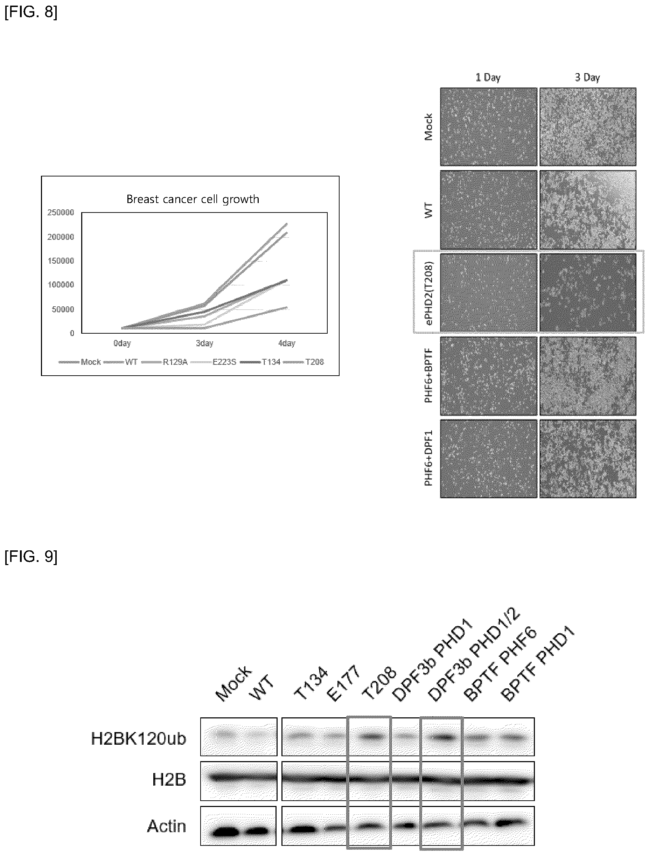

Cell proliferation was checked after gene delivery using lentivirus. It was found that ePHD2(T208) significantly inhibited cell proliferation in breast cancer cells (FIG. 8). A method to deliver the molecule to the cells was devised because it showed a significant reduction compared to other molecules that either promoted proliferation or had no effect.

The western blot data is shown in FIG. 9. In FIG. 9, row 1 represents H2BK120ub, row 2 represents H2B, and row 3 represents the housekeeping gene. After infection with the lentivirus, functional improvement through extension and swapping of the reader domain was confirmed by markers.

H2BK120Ub can be considered as a writer function of PHF6. Since it indicates increased activity, it is more likely to have certain improved abilities. Specifically, the marker was confirmed to increase in T208 and DPF3B PHD1/2, thereby confirming a potential as an improved candidate.

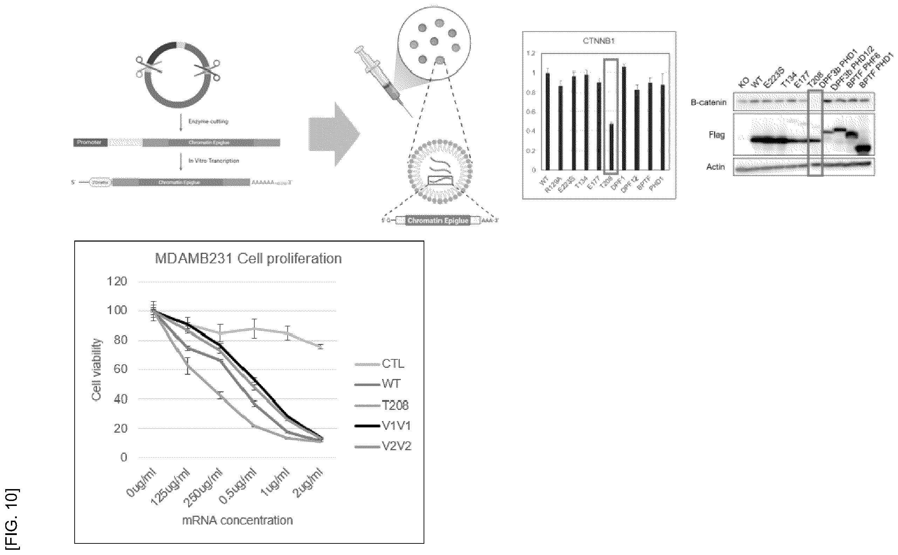

Furthermore, mRNA synthesized by in vitro transcription was used to express in breast cancer cells. As shown in FIG. 10, it was found that cell proliferation was inhibited under the conditions in which T208 was expressed. It was not as effective in a variant that induced a point mutation. The evidence that chromatin Epi-glue T208 can be used as a therapeutic agent in breast cancer was found.

### Example 5. Effect of ePHD2 (T208) in breast cancer (in vitro)

NGS miRNA-seq and ATAC-seq were used to identify the tumor regulatory mechanisms of T208(DR3). The miRNA-seq samples were transiently expressed in the breast cancer cell line MDA-MB-231 using the T208(DR3) transfection plasmid vector, and RNA was extracted 48 hours later using TRIzol. NGS analysis using the HiSeq250 platform was then performed. ATAC-seq samples were prepared in the same way as miRNA-seq samples, and libraries were constructed using the Illumina Tagment DNA Enzyme and Buffer Small Kit, followed by NGS analysis using the NovaSeq platform. The peaks of differentially expressed miRNAs that were changed in miRNA-seq analysis were compared with the peaks of miRNA-seq that were changed in ATAC-seq.

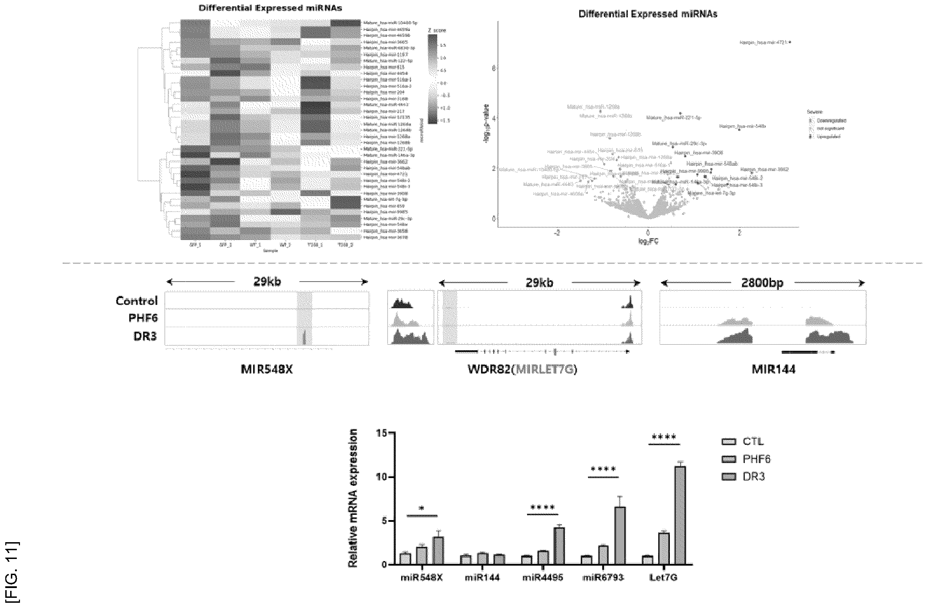

FIG. 11 shows that the expression of tumor suppressive miRNAs is increased in chromatin Epi-glue T208 (DR3) samples. It was confirmed that in chromatin Epi-glue T208, the inhibitory mechanism in breast cancer may be via miRNAs.

NGS RNA-seq and ATAC-seq were used to identify the tumor regulatory mechanisms of T208(DR3). Bulk RNA-seq samples were transiently expressed in breast cancer cell line MDA-MB-231 using T208(DR3) transfection plasmid vector under the same conditions as miRNA-seq samples, and RNA was extracted 48 hours later using TRIzol. NGS analysis using the NovaSeq platform was then performed. Significant genes were selected by analyzing differentially expressed genes, and peak calling of promoter and enhancer region was performed by ATAC-seq analysis and compared with RNA-seq data.

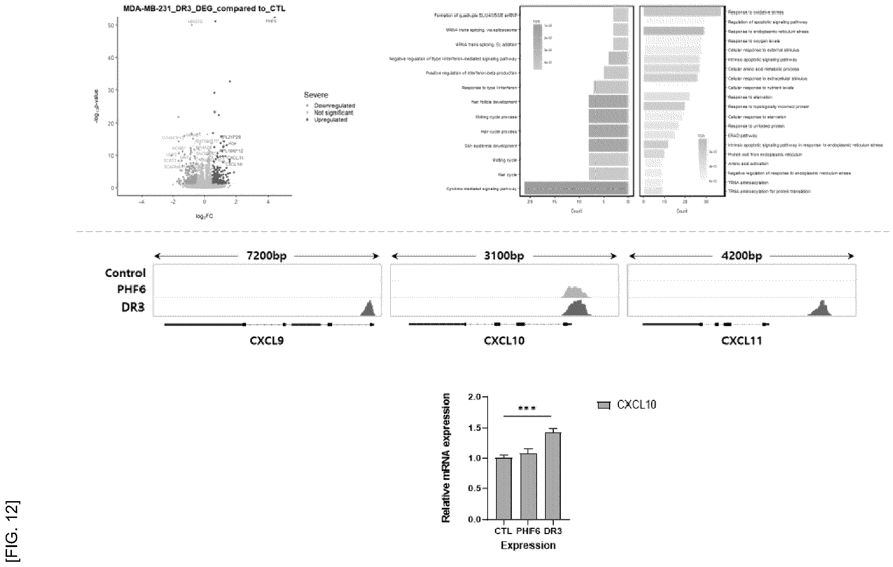

As shown in FIG. 12, the expression of chemokines among cytokines was increased in the chromatin Epi-glue T208 (DR3) sample. It was shown that chromatin Epi-glue T208 may have additional functions in breast cancer besides its inhibitory mechanism, such as promoting immunity.

### Example 6. Effect of ePHD2 (T208) in an in vivo xenograft model

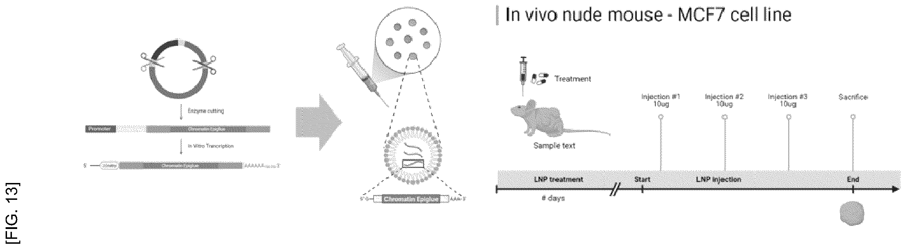

To examine in vivo efficacy, experiments were performed using an immunodeficient nude mouse model. MCF7 cell lines were seeded using subcutaneous injection as shown in FIG. 13. Four mice per condition were then injected with 10 ug of LNP or an equal volume of PBS every 2 days. After 2 weeks of monitoring, the mice were sacrificed to compare tumor size.

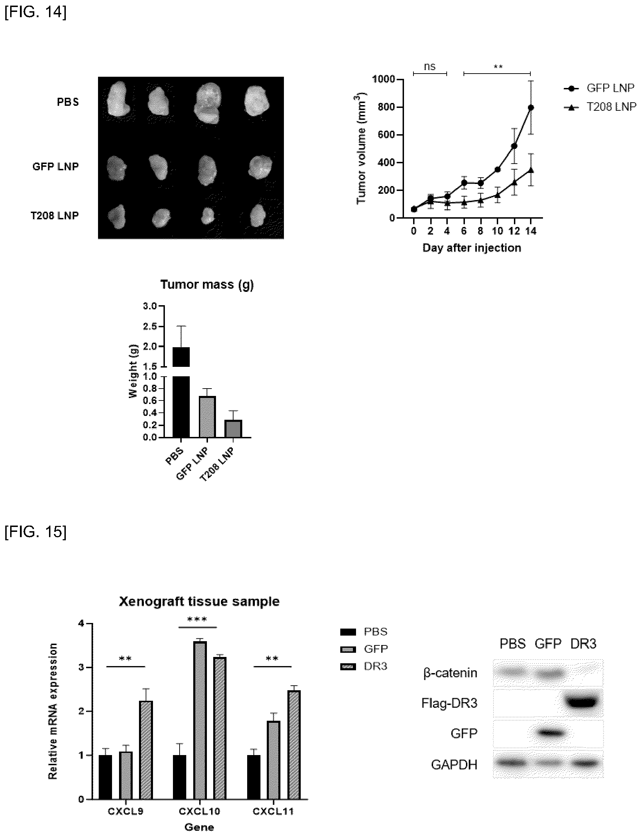

The efficacy of chromatin Epi-glue in an immunodeficient nude mouse xenograft model was verified and it was found that tumor growth was reduced compared to the control group (PBS group and GFP LNP group), as shown in FIG.14. This confirms that T208(DR3) can inhibit tumor formation in vivo.

In addition, tumor samples grown in Xenograft were used to identify target genes and proteins. To identify RNA, tumors removed from Xenograft were cut into approximately 3 mm pieces, and homogenized with 200 µl of TRIzol using a homogenizer, and RNA was extracted. The cDNA was then synthesized by reverse transcription reaction, and the expression of the expected chemokines was compared by quantitative PCR. Protein samples were added with 200 µl of EBC200 buffer, crushed using a homogenizer, and vortexed to dissolve sufficiently. The results were confirmed by SDS-PAGE and Western blot to determine the expression of the oncogenic protein β-catenin.

As shown in FIG. 15, the expression of genes related to chemokines increased in the T208(DR3) sample. The previously identified expression of β-catenin was also reduced.

### Example 6. Efficacy in cancers other than breast cancer

CCK8 assay in different cells was performed. Dojindo Cell counting kit-8 product was used. For floating cells, the cells were transfected in 6 wells and divided into 96 wells, and for adherent cells, the cells were placed in 96 well plates with media and mRNA reagents. Cell viability was measured in accordance with mitochondria activity on different days.

### (1) Blood cancer

The cell lines used in the experiments were Lymphoblast K562 from a CML patient and promyeloblast HL-60 cell line from an AML patient. In these cells, T208 showed the best cell growth inhibition. PHF6 also showed similar inhibitory behavior, which may indicate that mutations in PHF6 are associated with poor prognosis in leukemia.

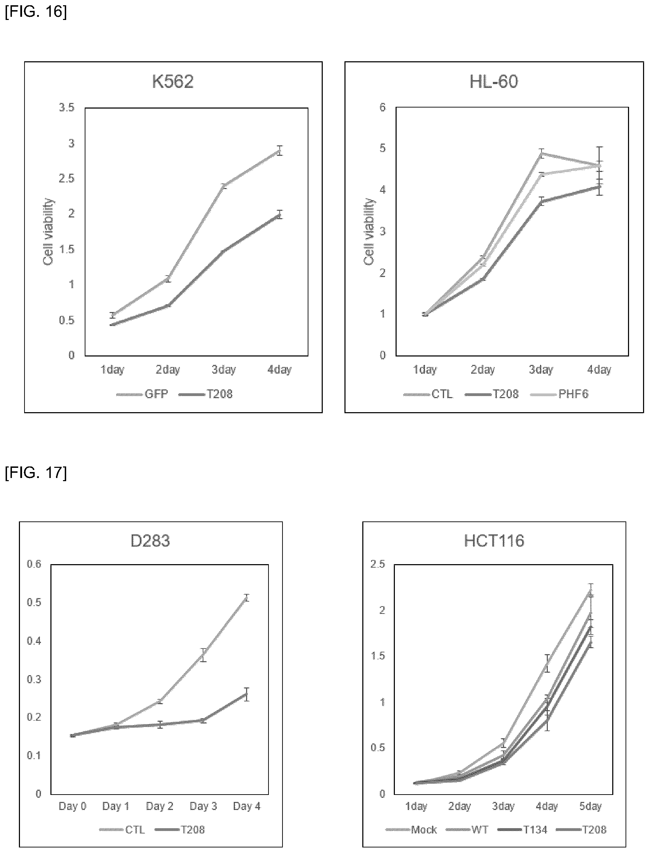

According to FIG. 16, there was a significant decrease in cell viability compared to the control group. Further increase in terms of expression of chemokines that attract immune cells in the RNA expression state would be meaningful in vivo.

### (2) Medulloblastoma and colon cancer

The cell lines used in the experiments were confirmed using epithelial cancer D283 from a medulloblastoma patient and epithelial cancer HCT116 cell line from a colorectal carcinoma patient.

As shown in FIG. 17, T208 also showed good cytostatic activity in these cells. In this cell line, the potential of this activity for other candidates was also observed.

### (3) Efficacy of the T208 variant

The cell lines used in the experiments were confirmed using lymphoblast RPMI6666 from a Hodgkin's disease patient, U-87 MG from a likely glioblastoma patient, and Daoy cell line from a desmoplastic cerebellar medulloblastoma patient.

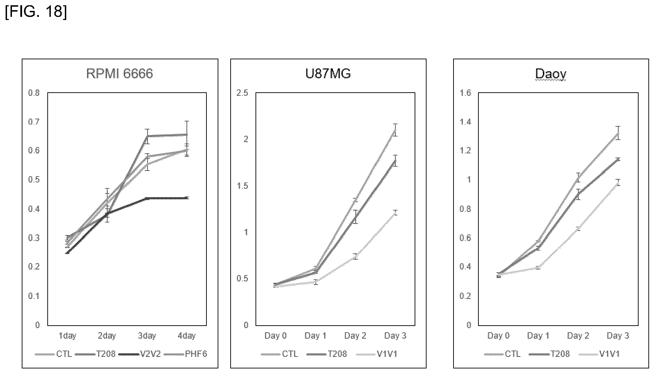

As shown in FIG. 18, T208 showed some efficacy in these cells, but less than in the previous cells. In addition, the V1V1 (#10 in FIG.6) and V2V2 (#11 in FIG.6) candidates, which induced two ePHD2 point mutations in T208(DR3), were expected to show better efficacy as their affinity was expected to have matured. In these cell lines, the potential usage of other candidates V1V1 and V2V2 was observed.

### Example 7. Efficacy of ePHD2(T208) in accordance with linker length

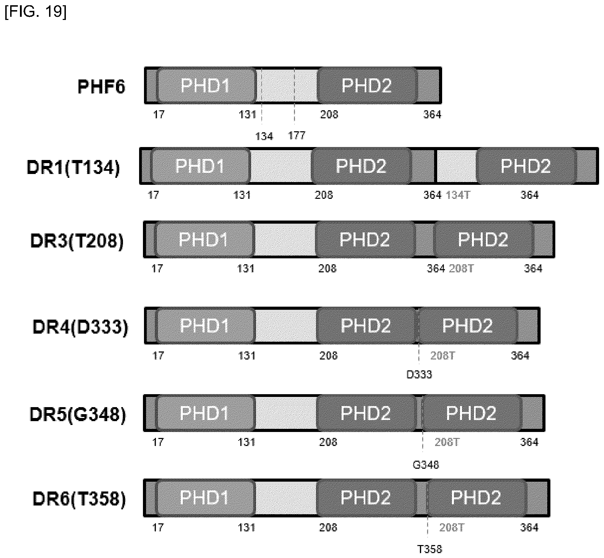

It was intended to determine the difference in efficacy based on the length of the linker between the two ePHD2 domains. Cell proliferation and migration experiments were performed to design and optimize the linker length as shown in FIG. 19. Equally seeded breast cancer cells were transiently transfected with T208(DR3) with different linker lengths. After sufficient expression was induced, equal numbers of cells were seeded in 96 well plates and compared for changes in confluency and wound scratch filling rate using Incucyte equipment.

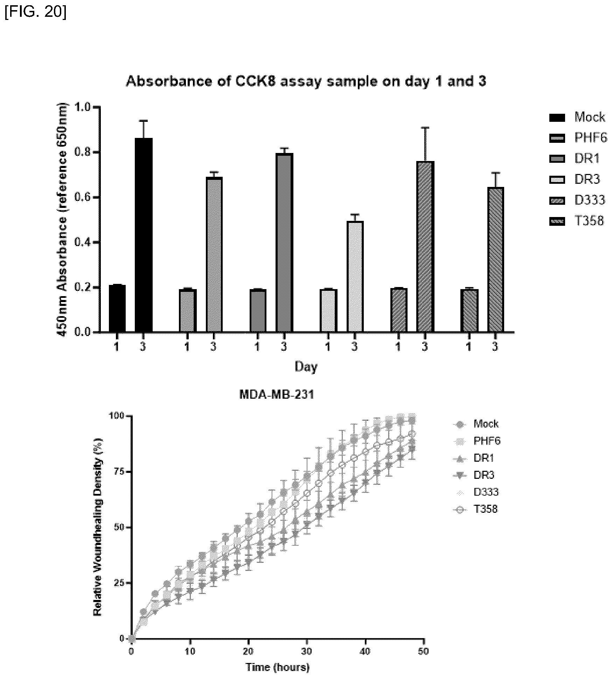

As shown in FIG. 20, the efficacy of ePHD2 domain linker length was validated and it was found that both cell proliferation assay (top of FIG. 20) and cell migration experiment (bottom of FIG. 20) showed good efficacy in inhibiting the progression of breast cancer in T208(DR3) samples.

While the foregoing has described in detail certain aspects of the present invention, it will be apparent to one of ordinary skill in the art that these specific descriptions are merely preferred examples and are not intended to limit the scope of the invention. Accordingly, the substantial scope of the invention will be defined by the appended claims and their equivalents.

## Claims

1. A polypeptide represented by the structure of D1-D2-D3, wherein:
(a) D1 is the PHD1 domain of PHF6 (PHD finger protein 6);
(b) D2 is the PHD2 domain of PHF6 or variants thereof; and
(c) D3 includes one or more selected from the group consisting of:
(i) D2;
(ii) a fragment of PHF6 containing D2;
(iii) PHD1 domain of double PHD fingers 3b (DPF3b);
(iv) PHD2 domain of DPF3b or variants thereof; and
(v) PHD1 domain and PHD2 domain of DPF3b, or variants thereof.

2. The polypeptide according to claim 1, wherein the D1 comprises D2 at the C-terminus, and the D2 comprises D3 at the C-terminus.

3. The polypeptide according to claim 1, wherein D1 comprises a sequence of SEQ ID NO: 2.

4. The polypeptide according to claim 1, wherein the PHD2 domain of D2 comprises a sequence of SEQ ID NO: 3.

5. The polypeptide according to claim 1, wherein the fragment of PHF6 comprising D2 in D3 is cleaved before one or more amino acid residues selected from the group consisting of positions 132 to 210 of the sequence of SEQ ID NO: 1.

6. The polypeptide according to claim 5, wherein the fragment of PHF6 comprising D2 is cleaved before an amino acid residue at position 134T or 177E of the sequence of SEQ ID NO: 1.

7. The polypeptide according to claim 1, wherein the PHD2 domain variant of PHF6 comprises one or more substitutions selected from the group consisting of SEQ ID NO: 1:
C212Y;
G218R;
I230T;
F231S;
N232K;
T253I;
Q270K or Q270R;
C280S;
I290V;
E293G or E293A;
Y301H;
C326R or C326S; and
S346R.

8. The polypeptide according to claim 1, comprising a sequence of SEQ ID NO: 6.

9. The polypeptide according to claim 1, wherein the PHD1 domain of DPF3b comprises a sequence of SEQ ID NO: 4; and/or
wherein the PHD2 domain of DPF3b comprises a sequence of SEQ ID NO: 5.

10. A nucleic acid encoding a polypeptide according to claim 1.

11. An mRNA encoding a polypeptide according to claim 1.

12. A cell into which the nucleic acid according to claim 10 is introduced.

13. A delivery vehicle comprising the mRNA according to claim 11.

14. The delivery vehicle according to claim 13, wherein the delivery vehicle comprises a nanoparticle, a liposome, a lipid nano-particle (LNP), or a vector.

15. A composition for the prevention or treatment of cancer comprising a polypeptide according to claim 1, a nucleic acid according to claim 10, an mRNA according to claim 11, a cell according to claim 12, or a delivery vehicle according to claim 13.
